# EUROPEAN PATENT SPECIFICATION

(11) **EP 1 670 513 B1**
(45) Date of publication and mention of the grant of the patent: **23.01.2013**
(21) Application number: 04809859.4
(22) Date of filing: 05.10.2004
(51) Int. Cl.: A61K 45/06, A61K 31/415, A61K 39/00, A61P 35/00, A61K 35/14

(54) **COX-2 INHIBITORS AND DENDRITIC CELLS FOR USE IN THE TREATMENT OF CANCER.**
COX-2-INHIBITOREN UND DENDRITISCHE ZELLEN ZUR VERWENDUNG IN DER KREBSBEHANDLUNG
INHIBITEURS DE COX-2 ET CELLULES DENDRITIQUES POUR L' UTILISATION DANS LE TRAITEMENT DU CANCER

(30) Priority: 06.10.2003 US 508945 P
(43) Date of publication of application: 21.06.2006
(73) Proprietor: Cedars-Sinai Medical Center, Los Angeles, California 90048-1865 (US)
(72) Inventor: YU, John, S., Los Angeles, CA 90048 (US); AKASAKI, Yasuharu, Los Angeles, CA 90024 (US)
(74) Representative: Teasdale, Nicola Joanne
(86) International application number: PCT/US2004/032669
(87) International publication number: WO 2005/037995

(56) References cited:
- WO-A2-03/035004
- HARIZI HEDI ET AL: "Prostaglandin E2 modulates dendritic cell function via EP2 and EP4 receptor subtypes." JOURNAL OF LEUKOCYTE BIOLOGY JUN 2003, vol. 73, no. 6, June 2003 (2003-06), pages 756-763, XP002454203 ISSN: 0741-5400
- EHTESHAM M ET AL: "Intratumoral dendritic cell vaccination elitics potent tumroicidal immunity against malignant glioma in rats" JOURNAL OF IMMUNOTHERAPY, LIPPINCOTT WILLIAMS & WILKINS, HAGERSTOWN, MD, US, vol. 26, no. 2, March 2003 (2003-03), pages 107-116, XP002983473 ISSN: 1524-9557
- YU J S ET AL: "Vaccination of malignant glioma patients with peptide-pulsed dendritic cells elicits systemic cytotoxicity and intracranial T-cell infiltration" CANCER RESEARCH, AMERICAN ASSOCIATION FOR CANCER RESEARCH, BALTIMORE, MD, US, vol. 61, no. 3, 1 February 2001 (2001-02-01), pages 842-847, XP002425827 ISSN: 0008-5472
- MÜLLER-DECKER KARIN ET AL: "The effects of cyclooxygenase isozyme inhibition on incisional wound healing in mouse skin." THE JOURNAL OF INVESTIGATIVE DERMATOLOGY NOV 2002, vol. 119, no. 5, November 2002 (2002-11), pages 1189-1195, XP002454204 ISSN: 0022-202X
- MATASIC R ET AL: "Cyclooxygenase-independent inhibition of dendritic cell maturation by aspirin." IMMUNOLOGY SEP 2000, vol. 101, no. 1, September 2000 (2000-09), pages 53-60, XP002454205 ISSN: 0019-2805
- SHENG H. ET AL.: 'Inhibition of human colon cancer cell growth by selective inhibition of cyclooxigenase-2.' J. CLIN. INHIB. no. 9, May 1997, pages 2254 - 2259, XP008049786
- ABIRU S. ET AL.: 'Aspirin and NS-398 inhibit hepatocycle growth factor-induced.' HEPATOLOGY. vol. 35, 2002, pages 1117 - 1124, XP002988613
- FERRANDINA G. ET AL.: 'Celecobix modulates the expression of cyclooxigenase -2,Ki67 apoptosis-related marker, and microvessel density in human cervical cancer.' CLIN.CANCER RES. vol. 9, 01 October 2003, pages 4324 - 4331, XP002988614
- ZITVOGEL L. ET AL.: 'Therapy of murine tumors with tumor peptide-pulsed dendritic cells: dependence on T cells, B7 constimulation, and helper cell-1 associated cytokines.' J. EXP.MED. vol. 183, January 1996, pages 87 - 97, XP002910558
- GATZA E. AND OKADA C.Y. ET AL.: 'Tumor cell lysate-pulsed dendritic cells are more effective than TCR id protein vaccines for active immunotherapy of T cell lymphoma.' J. IMMUNOL. vol. 169, 2002, pages 5227 - 5235, XP002988616

## Description

### FIELD OF THE INVENTION

The methods and reagents of the invention are used to treat or prevent diseases or conditions related to cell proliferation, such as cancer.

### BACKGROUND OF THE INVENTION

Cancer is the second leading cause of death in the United States, and over one million people are diagnosed with cancer each year. Approximately one out of every two American men and one out of every three American women will have some type of cancer during their lifetime. However, while substantial progress has been made in identifying some of the likely environmental and hereditary causes of cancer, the morbidity rates associated with this disease indicate a need for substantial improvement in the therapeutic interventions for cancer and related diseases and disorders.

The key to an effective immune response by the body, which will recognize and eradicate cancer cells, involves the activation of antigen-specific CD8+ cytotoxic T lymphocytes (CTLs) (De Plaen, E. et al., "Immunogenic (tum-) variants of mouse tumor P815: cloning of the gene of tum- antigen P91A and identification of the tum- mutation," Proc. Natl. Acad. Sci. U S A, Vol. 85, pp. 2274-2278 (1988); Van den Eynde, B. et al., "The gene coding for a major tumor rejection antigen of tumor P815 is identical to the normal gene of syngeneic DBA/2 mice," J. Exp. Med., Vol. 173, pp. 1373-1384 (1991); van der Bruggen, P. et al., "A gene encoding an antigen recognized by cytolytic T lymphocytes on a human melanoma," Science, Vol. 254, pp. 1643-1647 (1991)). The consensus on such an immune response is also supported by the evidence that antigenic peptides complexed to class I major histocompatibility (MHC) molecules have been observed as tumor-associated antigens (TAA) (Slingluff, C.L. et al., "Recognition of human melanoma cells by HLA-A2.1-restricted cytotoxic T lymphocytes is mediated by at least six shared peptide epitopes," J. Immunol., Vol. 150, pp. 2955-2963 (1993); Boon, T. et al., "Tumor antigens recognized by T lymphocytes," Annu. Rev. Immunol., Vol. 12, pp. 337-365 (1994); Fisk, B. et al., "Identification of an immunodominant peptide of HER-2/neu protooncogene recognized by ovarian tumor-specific cytotoxic T lymphocyte lines," J. Exp. Med., Vol. 181, pp. 2109-2117 (1995)). Studies have shown that tumor-specific CTLs must recognize TAA on the same antigen-presenting cells (APCs) that cross-present to CD4+ helper T (Th) cell epitopes in a cognate manner. This further indicates the requirement for epitope linkage between Th cells and CTLs for induction of potent anti-tumor immune responses (Bennett, S.R. et al., "Help for cytotoxic-T-cell responses is mediated by CD40 signaling," Nature, Vol. 393, pp. 478-480 (1998); Ridge, J.P. et al., "A conditioned dendritic cell can be a temporal bridge between a CD4+ T-helper and a T-killer cell," Nature, Vol. 393, pp. 474-478 (1998); Schoenberger, S.P. et al., "T-cell help for cytotoxic T lymphocytes is mediated by CD40-CD40L interactions," Nature, Vol. 393, pp. 480-483 (1998)). Given the importance of Th cell responses in anti-tumor immunity, the ability of APCs to skew the tumor-specific Th cells towards Th-subset-1 (Th1) state of activation may play a significant role in augmenting the efficacy of any systemically mounted tumoricidal immune response. An introduction to T lymphocytes and cell mediated immunity is found in Paul, Fundamental Immunology 3rd Edition, Raven Press (New York, N.Y. 1993), and the references cited therein.

One promising cancer therapy involves the use of dendritic cells (DCs) for induction of immunity. DCs are pivotal APCs equipped to capture and process TAA via dead cancer cells (Bennett, S.R. *et al.),* display high levels of class I and II MHC-antigenic peptide complexes with co-expression of co-stimulatory molecules, such as CD54, CD80 and CD86 (Banchereau, J. et al., "Dendritic cells and the control of immunity," Nature, Vol. 392, pp. 245-252 (1998)), and DCs are able to produce a high amount of interleukin (IL)-12 which polarizes Th cells toward Th1 (Koch, F. et al., "High level IL-12 production by murine dendritic cells: upregulation via MHC class II and CD40 molecules and downregulation by IL-4 and IL-10," J. Exp. Med., Vol. 184, pp. 741-746 (1996); Macatonia, S.E. et al., "Dendritic cells produce IL-12 and direct the development of Th1 cells from naive CD4+ T cells," J. Immunol., Vol. 154, pp. 5071-5079 (1995); Rissoan, M.C. et al., "Reciprocal control of T helper cell and dendritic cell differentiation," Science, Vol. 283, pp. 1183-1186 (1999)). Moreover, it has been shown that DCs express tumor necrosis factor (TNF) family ligands such as TNF, Lymphotoxin-α₁β₂, Fas ligand, and TNF-related apoptosis inducing ligand (TRAIL), and are fully equipped for an efficient direct apoptotic killing of cancer cells (Lu, G. et al., "Innate direct anticancer effector function of human immature dendritic cells. II. Role of TNF, lymphotoxin-alpha(1)beta(2), Fas ligand, and TNF-related apoptosis-inducing ligand," J. Immunol., Vol. 168, pp. 1831-1839 (2002)).

In clinical settings, high frequencies of natural and/or therapy-induced tumor-infiltrating DCs have been associated with better prognosis and/or regression of disease (Lynch, D.H. et al., "Flt3 ligand induces tumor regression and antitumor immune responses in vivo," Nat. Med., Vol. 3, pp. 625-631 (1997); Kikuchi, T. et al., "Intratumoral injection of dendritic and irradiated glioma cells induces anti-tumor effects in a mouse brain tumor model," Cancer Immunol. Immunother., Vol. 51, pp. 424-430 (2002); Ehtesham, M. et al., "Intratumoral dendritic cell vaccination elicits potent tumoricidal immunity against malignant glioma in rats," J. Immunother., Vol. 26, pp. 107-116 (2003)), providing evidence of their role in immune surveillance. However, there is evidence that cancers can evade immune surveillance by DCs, and the mechanism of escape is not completely understood.

DCs serve two important roles in the immune system by activating antigen-specific CTLs while at the same time maintaining CTL tolerance (Steinman, RM. et al., "The induction of tolerance by dendritic cells that have captured apoptotic cells," J. Exp. Med., Vol. 191, pp. 411-416 (2000); Verbovetski, I. et al., "Opsonization of apoptotic cells by autologous iC3b facilitates clearance by immature dendritic cells, down-regulates DR and CD86, and up-regulates CC chemokine receptor 7," J. Exp. Med., Vol. 196, pp. 1553-1561 (2002)). One explanation for this dual role of DCs is that the mechanism of DC-mediated CTL tolerance is caused by immature DCs, where the maturation arrest on DCs is caused by their uptake of apoptotic cells (Steinman, *R.M. et al.;* Verbovetski, I. *et al.;* Sauter, B. et al., "Consequences of cell death: exposure to necrotic tumor cells, but not primary tissue cells or apoptotic cells, induces the maturation of immunostimulatory dendritic cells," J. Exp. Med., Vol. 191, pp. 423-434 (2000)). However, DCs inoculated in the brain, which is considered an immunologically privileged site due to the lack of lymphatic drainage and the nature of the blood brain barrier (BBB) (Cserr, H.F. et al., "Cervical lymphatics, the blood-brain barrier and the immunoreactivity of the brain: a new view," Immunol. Today, Vol. 13, pp. 507-512 (1992)), migrated to the lymph nodes and induced anti-tumor immunity against brain tumors due to the stimulation of CTL activity (Kikuchi, T. *et al.;* Ehtesham, M. *et al.).* DC migration from periphery to lymphoid organs is associated with expression of chemokine receptor 7 (CCR7) which is one of the maturation markers of DCs (Yanagihara, S. et al., "EBI1/CCR7 is a new member of dendritic cell chemokine receptor that is up-regulated upon maturation," J. Immunol., Vol. 161, pp. 3096-3102 (1998)), indicating at least some tumor-infiltrating DCs do not fall into a maturation arrest after the DCs phagocytose apoptotic tumor cells. In addition, it has been shown that CTL tolerance is mediated by CD83+ mature DCs which are induced by ProstaglandinE₂ (PGE₂) stimulation, with the interface between the mature DCs and CD4+ Th cells being a critical checkpoint (Albert, M.L. et al., "Dendritic cell maturation is required for the cross-tolerization of CD8+ T cells," Nat. Immunol., Vol. 2, pp. 1010-1017 (2001); Kalinski, P. et al., "Prostaglandin E2 induces the final maturation of IL-12-deficient CD1a+CD83+ dendritic cells: the levels of IL-12 are determined during the final dendritic cell maturation and are resistant to further modulation," J. Immunol., Vol. 161, pp. 2804-2809 (1998)).

DCs containing tumor cells down-regulate the expression of co-stimulatory molecules such as CD86. The down-regulation of co-stimulatory molecules on DCs may be associated with the limited efficacy of anti-tumor immunity, but it is difficult to explain the mechanism of tumor generated escape from DC-mediated immune surveillance by this fact alone. It has been reported that tumor culture supernatant-exposed DCs lacked the capacity to produce IL-12 and did not acquire full allostimulatory activity (Kiertscher, S.M. et al., "Tumors promote altered maturation and early apoptosis of monocyte-derived dendritic cells," J. Immunol., Vol. 164, pp. 1269-1276 (2000); Pirtskhalaishvili, G. et al., "Cytokine-mediated protection of human dendritic cells from prostate cancer-induced apoptosis is regulated by the Bcl-2 family of proteins," Br. J. Cancer, Vol. 83, pp. 506-513 (2000)). One of the suppressive factors in tumor culture supernatant for DCs may be PGE₂ because of the evidence that PGE₂ induced mature DCs have impaired IL-12 production and those DCs are also associated with antigen-specific CTLs tolerance (Albert, M.L. *et al.;* Kalinski, P. *et al.).*

PGE₂ is a glioma-associated soluble factor that contributes to cellular immune suppression (Kuppner, M.C. et al., "Influence of PGE2- and cAMP-modulating agents on human glioblastoma cell killing by interleukin-2-activated lymphocytes," J. Neurosurg., Vol. 72, pp. 619-625 (1990); Sawamura, Y. et al., "In vitro prostaglandin E2 production by glioblastoma cells and its effect on interleukin-2 activation of oncolytic lymphocytes," J. Neurooncol., Vol. 9, pp. 125-130 (2000); Kokoglu, E. et al., "Prostaglandin E2 levels in human brain tumor tissues and arachidonic acid levels in the plasma membrane of human brain tumors," Cancer Lett., Vol. 132, pp. 17-21 (1998)), but its exact mechanism is unclear. PGE₂ is synthesized from arachidonic acid (AA), and the critical stage in PGE₂ formation is oxygenation of free AA by cyclooxygenase (COX) enzymes. There are two identified isoforms of COX enzymes, COX-1 and COX-2 (Leslie, C.C., "Properties and regulation of cytosolic phospholipase A2," J. Biol. Chem., Vol. 272, pp. 16709-16712 (1997)). COX-1 is constitutively present in many cell types, whereas COX-2 enzyme is inducible by several stimuli, such as interleukin (IL)-1-β and TNF-α (Smith, W.L. et al., "Prostaglandin endoperoxide H synthases (cyclooxygenases)-1 and -2," J. Biol. Chem., Vol. 271, pp. 33157-33160 (1996); DuBois, R.N. et al., "Regulation of eicosanoid production and mitogenesis in rat intestinal epithelial cells by transforming growth factor-alpha and phorbol ester," J. Clin. Invest., Vol. 93, pp. 493-498 (1994); Feng, L. et al., "Involvement of reactive oxygen intermediates in cyclooxygenase-2 expression induced by interleukin-1, tumor necrosis factor-alpha, and lipopolysaccharide," J Clin. invest., Vol. 95, pp. 1669-1675 (1995); Tamura, M. et al., "Interleukin-1beta elevates cyclooxygcnase-2 protein level and enzyme activity via increasing its mRNA stability in human endometrial stromal cells: an effect mediated by extracellularly regulated kinases 1 and 2," J. Clin. Endncrinol. Metab., Vol. 87, pp. 3263-3273 (2002)).

Many human malignant tumors reportedly overexpress COX-2, including colorectal cancer (Eberhart, C.E. et al., "Up-regulation of cyclooxygenase 2 gene expression in human colorectal adenomas and adenocarcinomas," Gastroenterology, Vol. 107, pp. 1183-1188 (1994)), lung cancer (Wolff, H. et al., "Expression of cyclooxygenase-2 in human lung carcinoma," Cancer Res., Vol. 58, pp. 4997-5001 (1998)), bladder cancer (Mohammed, S.I. et al., "expression of cyclooxygenase-2 (COX-2) in human invasive transitional cell carcinoma (TCC) of the urinary bladder," Cancer Res., Vol. 59, pp. 5647-5650 (1999)), and malignant glioma (Joki, T. et al.. "Expression of cyclooxygenase 2 (COX-2) in human glioma and in vitro inhibition by a specific COX-2 inhibitor, NS-398," Cancer Res., Vol. 60, pp. 4926-4931 (2000)). In addition, it has also been shown that COX-2 overexpression in glioma cells is associated with a clinically worse prognosis (Shono, T. et al., "Cyclooxygenase-2 expression in human gliomas: prognostic significance and molecular correlations," Cancer Res., Vol. 61, pp. 4375-4381 (2001)).

In spite of the advances in therapeutic treatments, including DC therapies, there exists a need in the art to understand the mechanisms of escape from immune surveillance. The elucidation of the mechanism of immunological tolerance against progressive cancers is required for induction of effective anti-cancer immunity, and one of the essential mechanisms is COX-2 overexpression in cancers.

### SUMMARY OF THE INVENTION

Described herein is a cyclooxygenase (COX)-2 inhibitor for use in the treatment of a cancer in a human, wherein the treatment includes the administration of a dendritic cell (DC) vaccine to said human.

In relation to the first aspect of the invention the COX-2 inhibitor may be used when the cancer to be treated by characterized by over expression of COX-2;
and may be used when the cancer is a glioma;
and may be used when the DC vaccine comprises from 10⁵ to 10⁷ DC;
and may be used when the COX-2 inhibitor is provided in a dose of from 0.1 to 10,000 mg per day;
and may be used when the COX-2 inhibitor is provided in a dose of from 1.0 to 1,000 mg per day;
and may be used when the cancer is selected from the group consisting of lung cancer, bladder cancer, colorectal cancer and brain cancer;
and may be used when the cancer is selected from the group consisting of gliomas, astrocytomas, ependymal, tumors, glioblastoma multiforme, and primitive neuroectodermal tumors;
and may be used when the COX-2 inhibitors is N5-398, celecoxib, rofecoxib, valdecoxib or meloxicam;
and may be used when the COX-2 inhibitor is present in an amount sufficient to selectively inhibit Prostaglandin E₂ (PGE₂) activity in the human;

According to a second aspect of the present invention there is provided a cyclooxygenase (COX)-2 inhibitor and dendritic cells (DCs) for use in the treatment of a cancer in a human;

In relation to the second aspect of the present invention the cyclooxygenase (COX)-2 inhibitor and dendritic cells (DCs) may be used when the cancer is characterized by expression of COX-2;
and may be used when the cancer is a glioma.

### BRIEF DESCRIPTION OF THE DRAWINGS

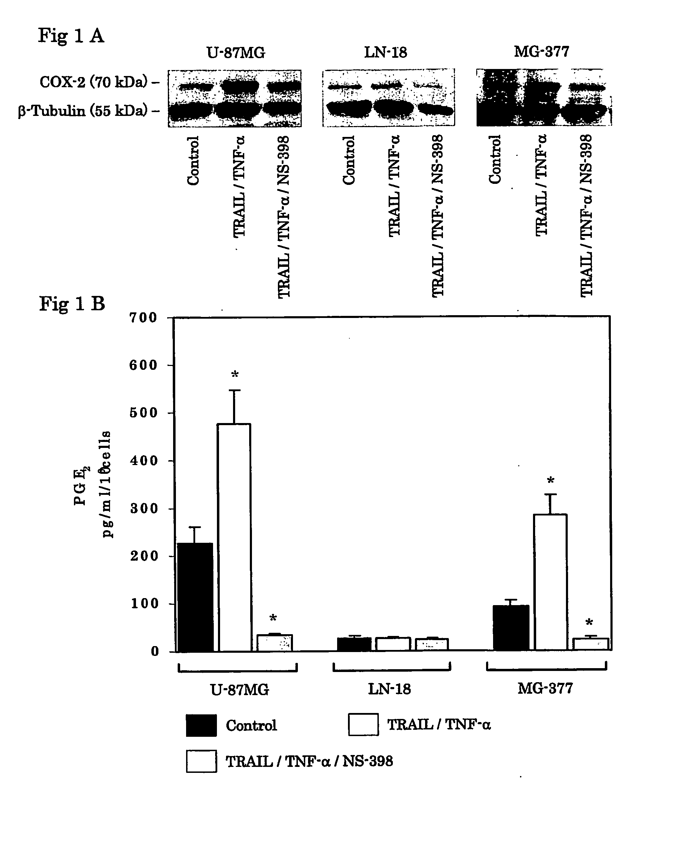

**FIGURE 1** depicts the relative levels of COX -2 and PGE₂ in human glioma cell lines including the U-87MG and LN-18 human glioma cell lines and primary cultured human glioma MG-377, in accordance with an embodiment of the present invention. Figure 1A depicts a Western blot analysis of COX-2 expression in human glioma. The analysis shows an increase in the expression level of COX-2 upon exposure to TNF family ligands and no change in COX-2 expression in the presence of COX-2 inhibitors. Figure 1B illustrates the results of an ELISA for PGE₂ in human glioma supernatant upon exposure to TNF family ligands and in the presence of COX-2 inhibitors.

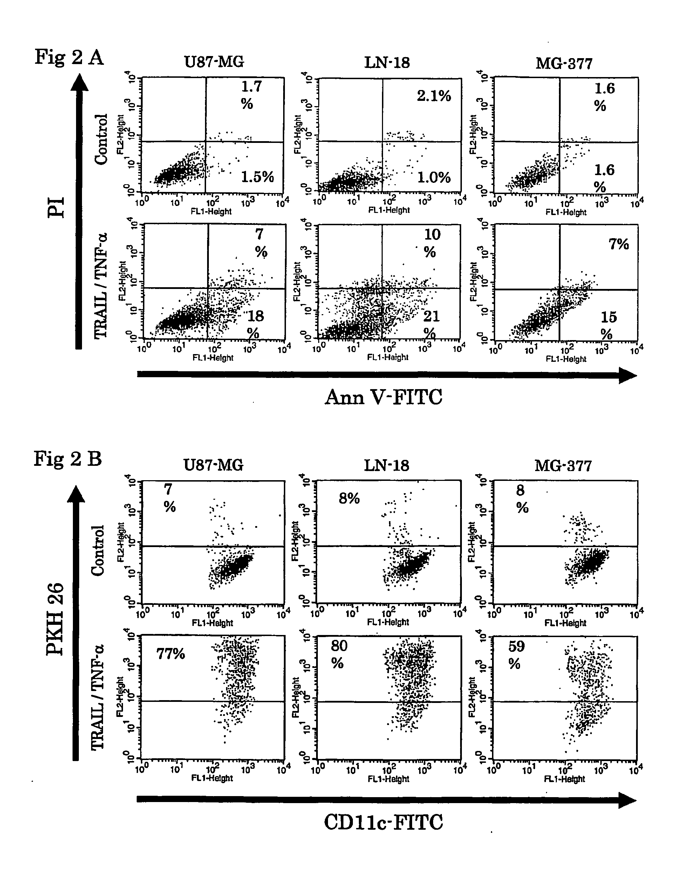

**FIGURE 2** depicts an analysis of apoptotic cell death in glioma cells using a Annexin V-FITC Apoptotis Detection Kit following treatment with DCs, in accordance with an embodiment of the present invention. Figure 2A shows a FACScan analysis indicating an increase in apoptotic death and cellular death following supplementation of TRAIL and TNF-a. Figure 2B shows a FACScan analysis indicating increased cell death in glioma causes an increase in phagocytosis by DCs as compared with each control.

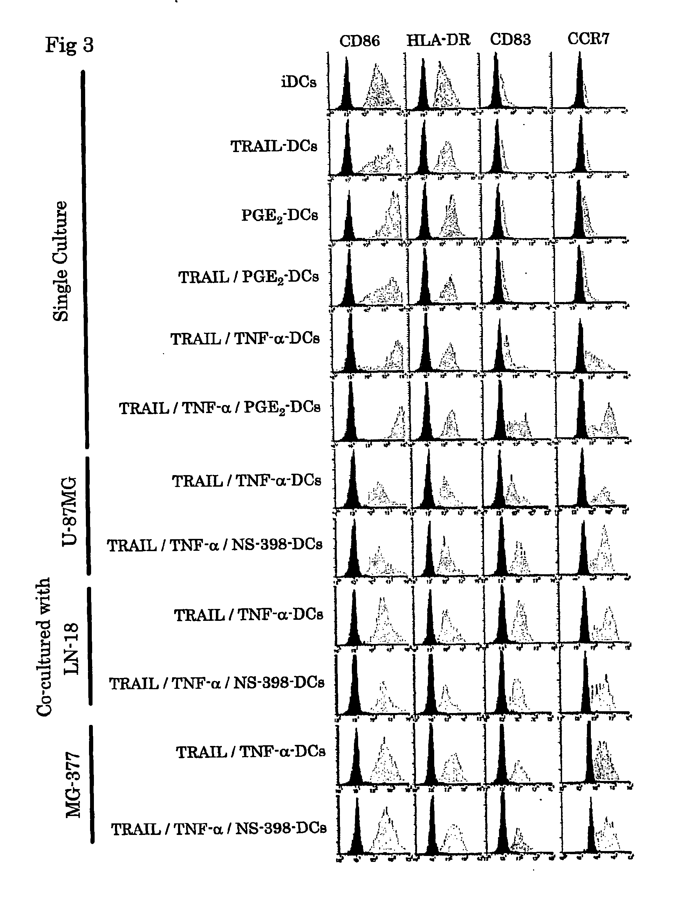

**FIGURE 3** depicts the relative expression of DC maturation markers in single culture and in co-culutre with U-87MG, LN-18 and MG-377 following treatment with TNF ligands and COX-2 inhibitor, in accordance with an embodiment of the present invention. The data indicates an upregulation of maturation markers CD83 and CCR7 upon stimulation of DCs with TNF ligands in single culture and the same results after co-culture and phagocytosis of glioma.

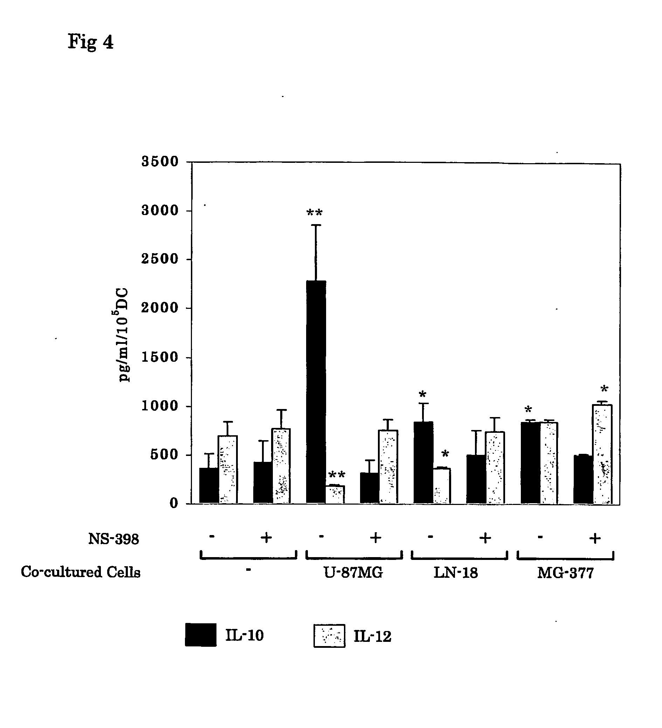

**FIGURE 4** depicts an ELISA analysis of the inhibition of COX-2 protein in glioma and its relative effective on the ratio of IL-10 and IL-12, in accordance with an embodiment of the present invention. Inhibition of COX-2 using NS-398 resulted in a downregulation of IL-10 and augmentation of IL-12.

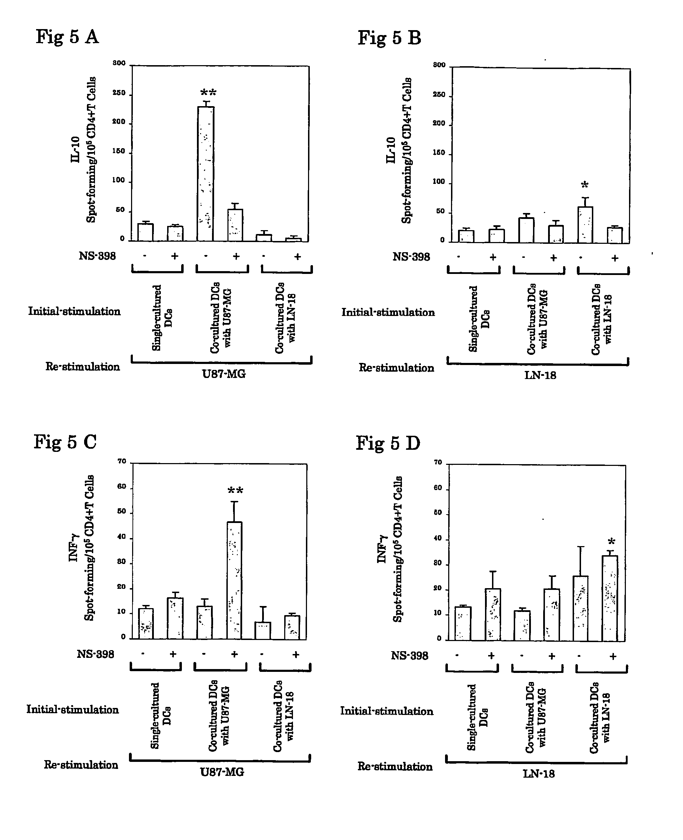

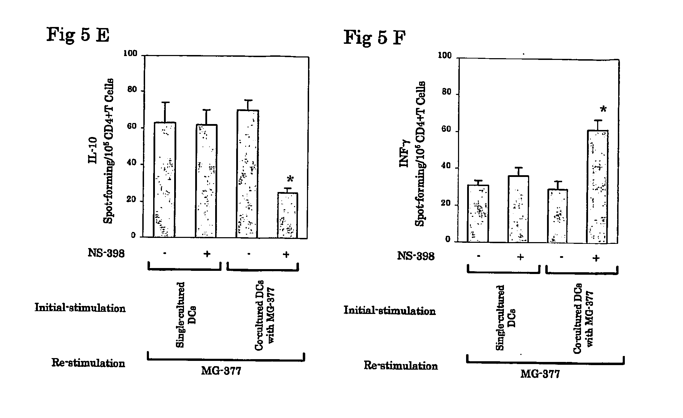

**FIGURE 5** demonstrates the capacity of COX-2 inhibitors in glioma for DC-mediating Th polarization to bias toward Th1, in accordance with an embodiment of the present invention. Figure 5A depicts the results of an ELISPOT assay showing a decrease in IL-10 expression in co-cultured DCs and glioma following treatment with NS-398. Figure 5B depicts the results of an ELISPOT assay showing a decrease in IL-10 expression in co-cultured DCs and glioma following treatment with NS-398. Figure 5C depicts the results of an ELISPOT assay showing an increase in interferon-gamma expression in co-cultured DCs and glioma following treatment with NS-398. Figure 5D depicts the results of an ELISPOT assay an increase in interferon-gamma expression in co-cultured DCs and glioma following treatment with NS-398. Figure 5E depicts the results of an ELISPOT assay showing a similar IL-10 expression in co-culture of primary glioma with DCs and a decrease in IL-10 following treatment with NS-398. Figure 5F depicts the results of an ELISPOT assay showing similar interferon-gamma expression in co-culture of primary glioma with DCs and a decrease in interferon-gamma following treatment with NS-398.

### DETAILED DESCRIPTION OF THE INVENTION

### A. DEFINITIONS

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994); March, Advanced Organic Chemistry Reactions, Mechanisms and Structure 4th ed., J. Wiley & Sons (New York, NY 1992), provides one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

"Alleviating" specific cancers and/or their pathology includes degrading a tumor, for example, breaking down the structural integrity or connective tissue of a tumor, such that the tumor size is reduced when compared to the tumor size before treatment. "Alleviating" metastasis of cancer includes reducing the rate at which the cancer spreads to other organs.

"Beneficial results" may include, but are in no way limited to, lessening or alleviating the severity of the disease condition, preventing the disease condition from worsening, curing the disease condition and prolonging a patient's life or life expectancy. The disease conditions may relate to or may be modulated by the central nervous system.

"Cancer" and "cancerous" refer to or describe the physiological condition in mammals that is typically characterized by unregulated cell growth. Examples of cancer include, but are not limited to, breast cancer, colon cancer, lung cancer, prostate cancer, hepatocellular cancer, gastric cancer, pancreatic cancer, cervical cancer, ovarian cancer, liver cancer, bladder cancer, cancer of the urinary tract, thyroid cancer, renal cancer, carcinoma, melanoma, head and neck cancer, and brain cancer; including, but not limited to, glioma, astrocytomas, ependymal tumors, glioblastoma multiforme, and primitive neuroectodermal tumors.

"Conditions" and "disease conditions," as used herein may include, but are in no way limited to any form of cancer.

"Curing" cancer includes degrading a tumor such that a tumor cannot be detected after treatment. The tumor may be reduced in size or become undetectable, for example, by atrophying from lack of blood supply or by being attacked or degraded by one or more components administered according to the invention.

"Cytokine" is a generic term for proteins released by one cell population which act on another cell as intercellular mediators. Examples of such cytokines are lymphokines, monokines, and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor (VEGF); integrin; thrombopoietin (TPO); nerve growth factors (NGFs) such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -γ; colony stimulating factors (CSFs) such as macrophage-CSF (M-CSF), granulocyte-macrophage-CSF (GM-CSF), and granulocyte-CSF (G-CSF); interleukins (ILs) such as IL-1, IL-1α, IL-2, IL-3, IL-4, IL-5, IL-6, IL-7, IL-8, IL-9, IL-10, IL-11, IL-12 and IL-13; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

A "dendritic cell" (DC) is an antigen presenting cell (APC) with a characteristic morphology including lamellipodia extending from the dendritic cell body in several directions. Several phenotypic criteria are also typical, including high levels of MHC molecules and co-stimulatory molecules, and a lack of markers specific for granulocytes, NK cells, B lymphocytes, and T lymphocytes, but can vary depending on the source of the dendritic cell. DCs are able to initiate antigen specific primary T lymphocyte responses *in vitro* and *in vivo,* and direct a strong mixed leukocyte reaction (MLR) compared to peripheral blood leukocytes, splenocytes, B cells and monocytes. Generally, DCs ingest antigen by phagocytosis or pinocytosis, degrade it, present fragments of the antigen at their surface and secrete cytokines.

"Exhibits" or "exhibiting" refers, generally, to the presence or display of something outwardly. For example, the terms may refer to the presence or display of a cell-surface marker or a transmembrane marker.

"Isolated" as used herein encompasses purified DCs that are substantially free of other cellular material, or culture medium.

"Mammal" as used herein refers to any member of the class *Mammalia,* including, without limitation, humans and nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be included within the scope of this term.

"Pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

"Therapeutic" as used herein refers to something tending to cure or restore to health. For example, something relating to the treatment of disease or disorders by remedial agents or methods and compounds that are used to treat specific diseases or medical conditions.

"Treatment" and "treating," as used herein refer to both therapeutic treatment and prophylactic or preventative measures, wherein the object is to prevent or slow down (lessen) the targeted pathologic condition or disorder even if the treatment is ultimately unsuccessful. Those in need of treatment include those already with the disorder as well as those prone to have the disorder or those in whom the disorder is to be prevented. In tumor (*e.g*., cancer) treatment, a therapeutic agent may directly decrease the pathology of tumor cells, or render the tumor cells more susceptible to treatment by other therapeutic agents, *e.g*., radiation and/or chemotherapy.

"Tumor," as used herein refers to all neoplastic cell growth and proliferation, whether malignant or benign, and all pre-cancerous and cancerous cells and tissues.

### B. DETAILED DESCRIPTION

The present invention is based on the surprising discovery of the mechanism of tumor-specific CTL tolerization induced by DCs infiltrating into COX-2 overexpressing gliomas. Human glioma cell lines U-87MG, LN-18 and primary cultured glioma (MG-377) were co-cultured with DCs as a human *in vitro* model to study tumor-infiltrating DCs. DCs efficiently phagocytosed apoptotic glioma cells and DCs were matured by TNF-α stimulation. However, a surplus of PGE₂ from COX-2 overexpressing glioma caused DCs to enhance IL-10 expression and to suppress IL-12 expression, which resulted in a bias by the tumor-infiltrating DCs of naïve CD4+Th cell development toward Th2. COX-2 inhibitor suppressed PGE₂ and tumor-infiltrating DCs polarized Th cells toward Th-subset-1 (Th1), indicating PGE₂ plays a major role in glioma generated CTL tolerance.

TNF-α stimulation of tumor-infiltrating DCs induced CD83+/CCR7+ mature DCs after they phagocytosed apoptotic glioma cells, supporting the notion that DCs can migrate to lymph nodes and present TAA to T cells. However, TNF-α and TRAIL, which induce DC maturation and tumor cell apoptosis, respectively, and both of which are equipped by DCs, up-regulated COX-2 expression in glioma cells. Thus, TNF-α and TRAIL necessarily induced PGE₂ overproduction from COX-2 overexpressing gliomas.

Infiltration of DCs into TNF-α /TRAIL treated glioma caused DCs to enhance IL-10 and to suppress IL-12, resulting in DCs that effectively bias naïve CD4+Th cell development toward Th2 in both allogenic and autologous models. Moreover, this tendency was strongest in the U-87MG glioma cell line, which produces the highest amount of PGE₂. The autologous model revealed that glioma patients already had a Th2 response against their tumors similar to what could be induced by the DCs co-cultured with autologous tumor. In contrast, the DCs infiltrating into COX-2 inhibited glioma polarized the Th cells toward Th1. Moreover, only by supplementing COX-2 overexpressing glioma cells with recombinant IL-12 was a potent therapeutic effect noted with DC-based glioma immunotherapy in the absence of COX-2 inhibitor (Akasaki, Y. et al., "Antitumor Effect of Immunizations With Fusions of Dendritic and Glioma Cells in a Mouse Brain Tumor Model," J. Immunother., Vol. 24, pp. 106-113 (2001)). The ability of tumors to skew the tumor-specific Th cells towards Th2 polarization plays a significant role in limiting the efficacy of CTL-based tumoricidal immune response and IL-12 plays a critical role for Th polarization toward Th1 in DC-mediating immunity of tumor-bearing hosts.

The relationship between PGE₂ in glioma products and the Th cell polarization mediated by the tumor-infiltrating DCs was confirmed using pTracer-COX-2 stable transfectant (available from Invitrogen; Carlsbad, CA), LN-18-COX-2 and pTracer-CMV2 stable transfectant, and LN-18-EP, where LN-18-EP expects to produce the same immune suppressants except PGE₂, as compared with LN-18-COX-2.

LN-18-COX-2 highly produced PGE₂, and completely affected DCs to bias Th cell development toward Th2. The addition of soluble PGE₂ into culture supernatant of LN-18-EP had the same effect on Th2 polarization as LN-18-COX-2. Furthermore, COX-2 inhibitor suppressed the PGE₂ that LN-18-COX-2 produced, thereby leading to tumor-infiltrating DCs polarizing the Th cells toward Th1. This further indicated that the PGE₂ in glioma product plays a major role in glioma generated tumor-specific CTL tolerance mediated by glioma-infiltrating DCs.

Treatment of cancer or cancerous tumors in a mammal is provided by administering to a mammal a therapeutically effective amount of a combination of a COX-2 inhibitor and a DC vaccine. While not wishing to be bound by any particular theory, it is believed that specific inhibitors of COX-2 may increase tumor response to DCs; thereby improving their efficacy.

As will be readily appreciated by those of skill in the art, the DC-based cancer vaccine and COX-2 inhibitor (or salt or derivative thereof) may be administered simultaneously. Alternatively, the COX-2 inhibitor may be administered before and/or concurrently with and/or following administration of the at least one therapeutic vaccination with the DC-based vaccine. In one embodiment of the present invention, a mammalian subject is administered a regular dosing regimen of the COX-2 inhibitor (*e.g*., daily, every other day, weekly, etc.) prior to and/or concurrently with and/or following the administration of the at least one therapeutic vaccination with the DC-based cancer vaccine. This dual treatment may be administered to a mammal to alleviate, and possibly cure, a host of disease conditions; particularly cancer, and more particularly, lung cancer, bladder cancer, colorectal cancer, or glioblastoma multiforme and other forms of brain cancer.

In various embodiments of the present invention the DC vaccine and COX-2 inhibitor may be included in a composition with one another. Alternatively, the DC vaccine and COX-2 inhibitor may be formulated for use as single agents and/or with additional COX-2 inhibitors or therapeutics as would be readily recognized by one of skill in the art.

There are various reasons why one might wish to administer a composition including both a DC vaccine and a COX-2 inhibitor of the present invention together, or why, in the alternative, one might wish to administer these components separately. Depending upon the particular DC vaccine and COX-2 inhibitor that one uses, a combination or a regimen of separate administrations might have superior characteristics as far as clinical efficacy, solubility, absorption, stability, toxicity and/or patient acceptability are concerned. It will be readily apparent to one of ordinary skill in the art how one can formulate a combined composition of any of a number of combinations of DC vaccines and COX-2 inhibitors of the present invention, or, alternatively, how one might establish a regimen of separate administrations. There are many strategies for doing either, any one of which may be implemented by routine experimentation, based on pharmacokinetics and other factors.

The cancer vaccine used in various embodiments of the instant invention may be selected from any DC-based cancer vaccine, and can be administered by routine methods. The DC may be autologous tumor antigen-presented DC that are "primed" *ex vivo* by conventional methods; for instance, the DC may be loaded with HLA-eluted peptides from cultured tumor cells or autologous tumor lysate. Alternatively, the DC may be delivered in an "unprimed" state and essentially primed *in vivo,* as described in U.S. patent application serial No. 10/251,148, filed September 20, 2002, the disclosure of which is incorporated by reference herein in its entirety. Use of "unprimed" DC may be particularly advantageous in instances where a tumor is surgically inoperable, where surgery is otherwise undesirable, or where no portion of the tumor can be retrieved for priming DC *ex vivo* against the tumor.

Briefly, "unprimed" DC include those that do not rely upon the acquisition of tumor tissue as a protein source, and the subsequent culturing therewith. In conventional methods, DC are primed *ex vivo.* Priming in this manner typically involves culturing the DC with the tumor cells against which they will subsequently be utilized; thereby providing the DC access to the tumor proteins and allowing the DC to process the associated antigens in preparation for presentation of the digested antigens to T-cells upon administration to a patient. However, in various embodiments of the present invention, DC may be delivered directly into a tumor bed or tumor region without first being primed *ex vivo;* the DC process the tumor antigens only *in vivo.*

DC suitable for use in accordance with various embodiments of the present invention may be isolated or obtained from any tissue in which such cells are found, or may be otherwise cultured and provided. For example, DCs may be derived from interstitial tissues such as the heart, kidney, gut and lung; DCs may be derived from Langerhan's cells such as the skin and mucous membrane; DCs may be derived from interdigitating dendritic tissues such as thymic medulla and secondary lymphoid tissue; and DCs may be derived from veiled cells of the lymph and blood. In particular, antigen-presenting DC may be used in accordance with the present invention. Such DC may be found, by way of example, in the bone marrow or human peripheral blood mononuclear cells (PBMCs) of a mammal, in the spleen of a mammal or in the skin of a mammal (*i.e.,* Langerhan's cells, which possess certain qualities similar to that of DC, may be found in the skin and may further be employed in conjunction with the present invention, and are included within the scope of DC used herein). For instance, in one embodiment of the present invention, bone marrow may be harvested from a mammal and cultured in a medium that promotes the growth of DC. GM-CSF, IL-4 and/or other cytokines, growth factors and supplements may be included in this medium. After a suitable amount of time, clusters of DC may be harvested and/or subcultured and subsequently harvested for use in a cancer vaccine.

Various other methods may be used to isolate the DCs of the present invention, as would be recognized by one of skill in the art. For example, DCs occur in low numbers in all tissues in which they reside, making isolation and enrichment of DCs a requirement. Any of a number of procedures entailing repetitive density gradient separation, fluorescence activated cell sorting techniques, positive selection, negative selection or a combination thereof are routinely used to obtain enriched populations or isolated DCs. Guidance on such methods for isolating DCs can be found in O'Doherty, U. et al., "Dendritic cells freshly isolated from human blood express CD4 and mature into typical immunostimulatory dendritic cells after culture in monocyte-conditioned medium," J. Exp. Med., Vol. 178, pp. 1067-1078 (1993); Young, J.W. and R.M. Steinman, "Dendritic cells stimulate primary human cytolytic lymphocyte responses in the absence of CD4+ helper T cells," J. Exp. Med., Vol. 171, pp. 1315-1332 (1990); Freudenthal, P.S. and R.M. Steinman, "The Distinct Surface of Human Blood Dendritic Cells, as Observed After an Improved Isolation Method," Proc. Nat. Acad. Sci. USA, Vol. 57, pp. 7698-7702 (1990); Macatonia, S.E. et al., "Suppression of immune responses by dendritic cells infected with HIV," Immunol., Vol. 67, pp. 285-289 (1989); Markowicz, S. and E.G. Engleman, "Granulocyte-macrophage colony-stimulating factor promotes differentiation and survival of human peripheral blood dendritic cells in vitro," J. Clin. Invest., Vol. 85, pp. 955-961 (1990); Mehta-Damani et al., "Generation of antigen-specific CD8+ CTLs from naive precursors," J. Immunol., Vol. 153, pp. 996-1003 (1994); and Thomas, R. et al., "Comparative accessory cell function of human peripheral blood dendritic cells and monocytes," J. Immunol., Vol. 151, pp. 6840-6852 (1993). One method for isolating DCs from human peripheral blood is described in U.S. Patent No. 5,643,786.

The DC-based cancer vaccine may be delivered to a recipient by any suitable delivery route, which may include, but is in no way limited to, injection, infusion, inoculation, direct surgical delivery, or any combination thereof. In one embodiment of the present invention, the DC-based cancer vaccine may be administered to a mammal by direct inoculation via stereotactic surgery; a standard inoculation procedure known to those of skill in the art of neurosurgery. Moreover, the vaccine may be administered to a tumor itself, to a physiologic region in close proximity to the tumor or to a remote location within a mammal with respect to the target tumor or tumors.

The DC-based cancer vaccine of the present invention may include "primed" or "unprimed" DC in a pharmaceutical carrier. Any conventional pharmaceutical carrier may be used in accordance with the present invention, and an appropriate carrier may be selected by one of skill in the art by routine techniques. In one embodiment of the present invention, the pharmaceutical carrier is saline, although other carriers may be utilized depending upon the desired characteristics of the cancer vaccine. For example, one may formulate a cancer vaccine differently in order to account for different delivery techniques for the vaccine, physiological differences among patients (*e.g*., sex, weight, age, etc.), or different types of tumors (*e.g*., brain, breast, lung, etc.), among other factors. The DC-based cancer vaccine administered to a mammal in accordance with the present invention may be delivered in combination with any of a variety of additional substances and compounds; for example, any suitable carrier, vehicle, additive, excipient, pharmaceutical adjunct, or other suitable product.

The quantity of DC appropriate for administration to a patient as a cancer vaccine to effect the methods of the present invention and the most convenient route of such administration may be based upon a variety of factors, as may the formulation of the vaccine itself. Some of these factors may include, but are in no way limited to, the physical characteristics of the patient (*e.g*., age, weight, sex, etc.), the physical characteristics of the tumor (*e.g*., location, size, rate of growth, accessibility, etc.), and the extent to which other therapeutic methodologies (*e.g*., chemotherapy, beam radiation therapy) are being implemented in connection with an overall treatment regimen. Notwithstanding the variety of factors one should consider in implementing the methods of the present invention to treat a disease condition, a mammal may be administered with from about 10⁵ to about 10⁷ DC in from about 0.05 mL to about 0.30 mL saline in a single administration, in one embodiment of the present invention. Additional administrations may be effected, depending upon the above-described and other factors, such as the severity of tumor pathology. In one embodiment of the present invention, from about one to about five administrations of about 10⁶ DC is performed at two-week intervals.

The COX-2 inhibitors used in connection with various embodiments of the present invention may exhibit anti-cancer properties. The COX-2 inhibitors may include, but are in no way limited to, NS-398 (available from Cayman Chemical; Ann Arbor, MI), celecoxib (available from Pfizer under the trade name "CELEBREX"; New York, NY), rofecoxib (available from Merck under the trade name "VIOXX"; Whitehouse Station, NJ), valdecoxib (available from Pfizer under the trade name "BEXTRA"), meloxicam (available from Boehringer Ingelheim under the tradename "MOBICOX"; Burlington, Ontario) and pharmaceutical equivalents, derivatives and salts, as well as other functionally related compounds as will be readily appreciated by those of skill in the art. Guidance as to additional COX-2 inhibitors is provided in the literature and generally available to practitioners in the art. *See, e.g.,* U.S. Patent No. 6,649,645 (describing the use of radiation with COX-2 inhibitors for the treatment of cancer). Alternatively, a nonselective COX inhibitor may be used in various embodiments of the invention in the form of a nonsteriodal anti-inflammatory drug (NSAID). A NSAID of the present invention may include, but is in no way limited to, R-etodolac, aspirin, diclofenac, diflunisal, etodolac, fenoprofen, floctafenine, flurbiprofen, ibuprofen, indomethacin, ketoprofen, meclofenamate, mefenamic acid, meloxicam, nabumetone, naproxen, oxaprozin, piroxicam, sunlindac, tenoxicam, tiaprofenic acid, tolmetin, and pharmaceutical equivalents, derivatives and salts, as well as other functionally related compounds, although numerous other NSAIDs may be used, as will be readily appreciated by those of skill in the art. For example, guidance as to particular NSAIDS is provided in the literature and generally available to practitioners in the art. *See, e.g.,* U.S. Patent No. 6,761,913 (describing the use of celery seed extracts and additional NSAIDS for the treatment of inflammation), and U.S. Patent No. 6,759,056 (describing a transdermal delivery system incorporating numerous NSAIDs).

In various embodiments of the present invention, the COX-2 inhibitor can be formulated in a pharmaceutical composition. Such a composition may be administered orally, parenterally, by inhalation spray, rectally, or topically in dosage unit formulations containing conventional nontoxic pharmaceutically acceptable carriers, adjuvants, and vehicles as desired. Other routes of administrations are well within the level of knowledge in the art, and such routes are considered within the ambit of the present invention. Moreover, topical administration can also involve the use of transdermal administration such as transdermal patches or iontophoresis devices. The term "parenteral," as used herein, includes subcutaneous injections, intravenous, intramuscular, intrasternal injection, or infusion techniques. One skilled in the art will recognize many additional formulations or equivalents to those described above, which could be used in the practice of the present invention.

Injectable preparations such as sterile injectable aqueous or oleaginous suspensions can be formulated using suitable dispersing or wetting agents and suspending agents. Acceptable nontoxic diluents or solvents can be used in the sterile injectable preparation as a solution or suspension for parenteral administration, for example, as a solution in 1,3-butanediol. Example diluents and solvents include, but are in no way limited to, water, Ringer's solution, and isotonic sodium chloride solution. Fixed oils are also conventionally employed as a sterile solvent or suspending medium; for example, synthetic mono- or diglycerides. In addition, fatty acids such as oleic acid can be used in the preparation of injectables, as well as dimethyl acetamide, surfactants including ionic and non-ionic detergents, and polyethylene glycols. Furthermore, mixtures of any of the above listed elements may be used in preparation of the pharmaceutical composition.

Oral administration can include capsules, tablets, pills, powders, and granules. In addition, liquid dosage forms for oral administration can include pharmaceutically acceptable emulsions, solutions, suspensions, syrups, and elixirs containing inert diluents commonly used in the art, such as water. One or more adjuvants appropriate to the indicated route of administration such as wetting agents, emulsifying and suspending agents, and sweetening, flavoring, and perfuming agents. For example, if administered orally, a COX-2 inhibitor could be mixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets can contain a controlled-release formulation as can be provided in a dispersion of active compound in hydroxypropylmethyl cellulose. In the case of capsules, tablets, and pills, the dosage forms can also comprise buffering agents such as sodium citrate, magnesium or calcium carbonate or bicarbonate. Tablets and pills can additionally be prepared with enteric coatings.

Formulations for parenteral administration can be in the form of aqueous or nonaqueous isotonic sterile injection solutions or suspensions. These solutions and suspensions can be prepared from sterile powders or granules having one or more of the carriers or diluents mentioned for use in the formulations for oral administration. A contemplated COX-2 inhibitor compound can be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well known and would be recognized by one of skill in the art.

In any of the embodiments of the invention, the DC vaccines and COX-2 inhibitors can be administered in combination with other appropriate therapeutic treatments. For example, the DC vaccine and COX-2 inhibitor of the present invention may be administered in addition to an established therapy, such as chemotherapy, radiation treatment or any other therapy known in the art to treat a disease condition. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects.

The determination of a therapeutically effective dose is well within the capability of those skilled in the art. A "therapeutically effective" dose refers to that amount of active ingredient which increases or decreases the effects of a disease condition relative to that which occurs in the absence of the therapeutically effective dose. Therapeutic efficacy and toxicity, *e.g.*, ED₅₀ (the dose therapeutically effective in 50% of the population) and LD₅₀ (the dose lethal to 50% of the population), can be determined by standard pharmaceutical procedures in cell cultures or experimental animals. The dose ratio of toxic to therapeutic effects is the therapeutic index, and it can be expressed as the ratio, LD₅₀/ED₅₀. Furthermore, the data obtained from cell culture assays and animal studies is used in formulating a range of dosage for human use, which can be readily tended to by one of ordinary skill in the art without undue experimentation. The dosage contained in such compositions may be selected so as to be within a range of circulating concentrations that include the ED₅₀ with little or no toxicity. The dosage varies within this range depending upon the dosage form employed, sensitivity of the patient, and the route of administration.

The appropriate dosage of the COX-2 inhibitor of the invention may depend on a variety of factors. Such factors may include, but are in no way limited to, a patient's physical characteristics (*e.g*., age, weight, sex), whether the COX-2 inhibitor is being administered in a composition or as part of a combination therapy, the type of disease condition being treated, the progression (*i.e*., pathological state) of the cancer or other disease condition, and other factors that may be recognized by one skilled in the art. Furthermore, the therapeutically effective doses can be estimated initially either in cell culture assays or in animal models, usually mice, rabbits, dogs, or pigs. The animal model also can be used to determine the appropriate concentration range and route of administration. Such information can then be used to determine useful doses and routes for administration in humans. However, the exact dosage will be determined by the practitioner, in light of factors related to the subject that requires treatment. Notwithstanding the variety of factors one should consider in implementing the methods of the present invention to treat a disease condition, a mammal may be administered from about 0.1 mg to about 10,000 mg per day of the COX-2 inhibitor, in one embodiment of the present invention. In one embodiment of the present invention, from about one to about three administrations of about 1.0 to about 1,000 mg per day COX-2 inhibitor is performed each day. Alternatively, the COX-2 inhibitor may be administered every other day, weekly, or on any other suitable periodic basis and at any other dose, as will be readily appreciated by one of skill in the art.

In another embodiment of the present invention, a kit is included, having a quantity of at least one therapeutic DC vaccine, at least one therapeutic COX-2 inhibitor and instructions for their use in treating a disease condition. The exact nature of the components configured in the inventive kit depends on its intended purpose and on the particular methodology that is employed. For example, some embodiments of the kit are configured for the purpose of alleviating, curing or treating cancer in a subject. In one embodiment, the kit is configured particularly for the purpose of delivering therapeutic treatments to gliomas in a human subject.

Instructions for use may be included with the kit. "Instructions for use" typically include a tangible expression describing the steps for inoculating a subject with DCs and administering a COX-2 inhibitor and/or for using the same in a therapeutic system. Optionally, the kit also contains other useful components, such as diluents, buffers, pharmaceutically acceptable carriers, specimen containers, syringes, stents, catheters, pipetting or measuring tools, and the like.

The materials or components assembled in the kit can be provided to the practitioner stored in any convenient and suitable way that preserves their operability and utility. For example, the components can be in dissolved, dehydrated, or lyophilized form. They can be provided at room, refrigerated, or frozen temperatures.

The components are typically contained in suitable packaging material(s). As employed herein, the phrase "packaging material" refers to one or more physical structures used to house the contents of the kit. The packaging material is constructed by well known methods, preferably to provide a sterile, contaminant-free environment. The packaging materials employed in the kit are those customarily utilized in the field. As used herein, the term "package" refers to a suitable solid matrix or material such as glass, plastic, paper, foil, and the like, capable of holding the individual kit components. Thus, for example, a package can be a glass vial used to contain suitable quantities of DCs and COX-2 inhibitors. The packaging material generally has an external label which indicates the contents and/or purpose of the kit and/or its components.

### EXAMPLES

The following examples are typical of the procedures that may be used to administer a combination therapy of DCs and COX-2 inhibitor for the treatment of cancer. Modifications of these examples will be readily apparent to those skilled in the art who seek to treat patients whose condition differs from those described herein. Statistical results were analyzed using the student's T test.

### EXAMPLE 1

### Tumor Cells, Media and Reagents

Tumor cells, including U-87MG human glioma cell line (obtained from American Type Culture Collection; Rockville, MD), LN-18 cell line (obtained from Dr. Erwin Van Meier, Emory University; Atlanta, GA), and primary cultured human glioma MG-377 (obtained from surgical specimen of a glioblastoma patient at neurosurgical institute, Cedars-Sinai Medical Center; Los Angeles, CA), were maintained at 37°C and 5% CO₂ in Dulbecco's Modified Eagles Medium (available from Sigma; St. Louis, MO; hereinafter "DMEM") supplemented with 10% heat-inactivated fetal bovine serum (available from Sigma; hereinafter "FBS"), 2mM glutamate, 10mM HEPES (available from Sigma), 100U/ml penicillin (available from Sigma), 100µg/ml streptomycin (available from Sigma). 100% dimethyl sulfonyl oxygen (available from Sigma; hereinafter "DMSO") dissolved NS-398 (selective COX-2 inhibitor; obtained from Cayman Chemical) was used at concentrations of 10 µM. Recombinant human TNF-α (obtained from BioSource International; Camarillo, CA) was used at a concentration of 20 ng/ml and TRAIL (obtained from PeproTech Inc.; Rocky Hill, NJ) was used at a concentration of 300 ng/ml.

### EXAMPLE 2

### COX-2 cDNA Plasmid and Transfection

The COX-2 cDNA was isolated from the pSG5-COX-2 plasmid, which contains a full-length COX-2 cDNA in the pSG expression vector (obtained from Dr. Richard Kulmacz, University of Texas Medical School; Houston, TX) by EcoRI and XbaI digestion. COX-2 expression plasmid, designated pTracer-COX-2, was constructed by inserting COX-2 cDNA between the EcoRI and XbaI sites on pTracer-CMV2 expression vector (obtained from Invitrogen), which is available to express green fluorescent protein (GFP) fused to the selectable marker Zeocin. Lipofectamine 2000 (obtained from Invitrogen) and Plus Reagent (obtained from Invitrogen) were used for transfection to LN-18 according to the manufacture's protocols. The transfected cells of pTracer-COX-2, and the empty plasmid, pTracer-CMV2, were selected by Zeocin (obtained from Invitrogen), and stable transfectants were established (LN-18-COX-2, LN-18-EP).

### EXAMPLE 3

### Detection of Apoptotic Cell Death

Tumor cells were treated with TNF-α and TRAIL for 24 hours. Apoptotic death was detected using Annexin V-FITC Apoptosis Detection Kit I (obtained from Phamingen; San Diego, CA). Cells were stained with Annexin V-FITC (Ann V) and propidium iodide (PI) according to the manufacture's protocol. Early stage of apoptosis was defined by Ann V⁺/ PI⁻ staining as analyzed by fluorescence multicolor flow cytometry (obtained from Becton Dickinson Immunocytometry Systems; San Jose, CA; hereinafter "FACScan").

### EXAMPLE 4

### Extraction of Cell Samples for Western Blot

Samples were extracted with buffer containing 1% Triton X-100, 150 mM NaCl, 50 mM Tris (hydroxymethyl aminomethane) (pH 7.5) and 1 mM phenylmethylsulfonyl fluoride (PMSF). The cell debris was removed by centrifugation at 14,000 g for 20 minutes and the supernatant was subjected to SDS polyacrylamide gel electrophoresis (SDS-PAGE) and loaded on a 7.5% polyacrylamide gel (obtained from Bio-Rad; Hercules, CA). Electrophoretic transfer to nitrocellulose membranes (obtained from Amersham Biosciences; Piscataway, NJ) was followed by immunoblotting with a mouse IgG1 anti-COX-2 antibody (obtained from Phamingen) or a mouse IgG1 anti-beta-tublin (obtained from Sigma). This was followed by hybridization with a peroxidase-linked anti-mouse IgG antibody (obtained from Amersham Bioscience). The signal was detected by chemiluminescence using ECL detection system (obtained from Amersham Bioscience) and Hyperfilm ECL (obtained from Amersham Bioscience).

### EXAMPLE 5

### Isolation and Enrichment of Dendritic Cells and Lymphocytes

Human PBMCs were separated from peripheral blood of a healthy donor and the aforementioned glioblastoma patient using Ficoll-Hypaque density centrifugation. PBMC were resuspended in X-Vivo 15 serum-free medium (obtained from Cambrex; Santa Rosa, CA) and allowed to adhere to 24-well culture plate at 37°C. The non-adherent cells were removed after 2 hours. CD4+ and CD8+ cells were isolated from these non-adherent cells with MiniMACS cell separation unit (obtained from Milenyi Biotec, Auburn, CA) and microbeads conjugated mouse monoclonal anti-human CD4 and CD8 antibody (obtained from Milenyi Biotec). CD4+ and CD8+ cells were used for mixed lymphocyte reaction (MLR) described later. The adherent cells were subsequently cultured in X-Vivo 15 serum-free medium. 20 ng/ml GM-CSF (obtained from BioSource International) and 10 ng/ml IL-4 (obtained from BioSource International) were added to the cultures on days 0, 2, and 4. After 7-day culture, the floating cells were transferred to fresh plates with fresh X-Vivo 15 medium as immature DCs (iDCs). Several iDCs were cultured with 300 ng/ml TRAIL and/or 0.1 µM PGE₂ (obtained from Sigma) and/or 20 ng/ml TNF-α for 16 hours (TRAIL-DCs, PGE₂-DCs, TRAIL/PGE₂-DCs, TRAIL/ TNF-α -DCs, TRAIL/ TNF-α /PGE₂-DCs).

### EXAMPLE 6

### Co-culture of Dendritic Cells and Glioma Cells

Glioma cells were plated to 6-well culture plate at density of 1x10⁶ cells/well/2 ml with fresh X-Vivo 15 medium. Several glioma cells were pre-treated with the COX-2 inhibitor NS-398 for 8 hours. The NS-398 treated glioma cells were cultured with TNF-α, TRAIL, and NS-398. The other cells were cultured with TNF-α and TRAIL. After 24 hours culture with these agents, iDCs were put into the wells at the density of 3x10⁵ cells/well, and were co-cultured with glioma cells for 16 hours. Several iDCs were cultured with TNF-α /TRAIL or TNF-α /TRAIL/NS-398 without tumor cells. Human glioma cell lines (U-87MG and LN-18) and stable transfectants (LN-18-COX-2 and LN-18-EP) were co-cultured with healthy donor iDCs, and MG-377 were co-cultured with autologous iDCs.

### EXAMPLE 7

### Flow Cytometric Analysis

Monoclonal mouse anti-human antibodies of PE-conjugated CD83, CD86, HLA-DR (obtained from Phamingen), FITC-conjugated CD11c (obtained from BioSource International), and unconjugated CCR7 (R&D Systems; Minneapolis, MN) were used for cell surface analysis of DCs. PE-conjugated F(ab')₂ goat anti-mouse immunoglobulins (obtained from DAKO Cytomation; Carpinteria, CA) was used for secondary antibody. Mouse IgG1 and IgG2a (obtained from Phamingen) were used for isotype control. DCs were stained with anti-CD86, HLA-DR, CD83, and CCR7 antibodies before staining with anti-CD11c antibody, and CD11c+ DCs were isolated with MiniMACS cell separation unit and microbeads-conjugated anti-FITC antibody (obtained from Milenyi Biotec).

Tumor cells were dyed red with PKH-26 (obtained from Sigma) before co-culturing with iDCs. DCs were stained with anti-CD11c-FITC antibody after 16 hours co-culture, and were isolated as mentioned above. Isolated CD11c+ DCs were analyzed by FACScan where double positive cells indicate uptake of the tumor cells by DCs.

### EXAMPLE 8

### ELISA Analysis

PGE₂ ELISA kit (obtained from Cyman Chemical; Ann Arbor, MI) was used according to the manufacture's protocol for measurement in the supernatants of glioma cell cultures. IL-10 and IL-12 p70 ELISA kits (obtained from Phamingen) were used according to the manufacture's protocols for measurement in the supernatants of cultures of tumor cells or isolated CD11c+ DCs. CD40-CD40L interaction is a main trigger of IL-12 production by DCs (Cella, M. et al., "Ligation of CD40 on dendritic cells triggers production of high levels of interleukin-12 and enhances T cell stimulatory capacity: T-T help via APC activation," J. Exp. Med., Vol. 184, pp. 747-752 (1996)). Based on this evidence, cells were co-cultured with NIH-CD40L cells (obtained from Dr. Gang Zeng, National Cancer Institute, NIH, MD; the ratio of NIH-CD40L : DCs = 1 : 1) for 16 hours, and the supernatants of co-cultures were used as the samples. The lower detection limit of each kit was 7.8 pg/ml. All samples and standards were run in duplicate.

### EXAMPLE 9

### Mixed Lymphocyte Reaction and ELISPOT Analysis

CD11c+ DCs were co-cultured with autologous CD4+ and CD8+ lymphocytes with fresh X-Vivo 15 medium for 7 days (the initial stimulation; the ratio of DCs:lymphocytes = 1:40∼50) in 6-well culture plate. The lymphocytes were transferred to fresh plates with fresh X-Vivo 15 medium and were re-stimulated with 1x10⁵ glioma cells for 48 hours. After re-stimulation, only CD4+ cells were isolated again as mentioned above, and were re-suspended in fresh X-Vivo 15 medium.

Dual human INF-γ/IL-10 ELISPOT PVDF-enzymatic kit (obtained from Cell Sciences; Norwood, MA) and PVDF-bottomed 96-well plate (obtained from Cellular Technology; Cleveland, OH) were used for ELISPOT assay of the isolated CD4+ T cells. CD4+ T cells (1x10⁵ cells/100µl/well) were plated to INF-γ- or IL-10-capture antibody coated wells and were cultured at 37°C and 5% CO₂ for 20 hours. All samples were run in duplicate. Spot-forming was analyzed by Alpha Imager Spot-reading System (obtained from Alpha Innotech; San Leandro, CA).

### EXAMPLE 10

### Human Glioma Cells Overexpress COX-2 Protein and Highly Produce PGE₂

Expression of COX-2 protein in human glioma cells was examined by Western blot (Fig. 1A). COX-2 protein was detected in U-87MG, LN-18, and MG-377, and the treatment with TRAIL and TNF-α highly induced COX-2 expression in U-87MG and MG-377. COX-2 inhibitor did not extinguish the COX-2 expression. There was no major difference of COX-2 expression in each group of LN-18.

PGE₂ in the culture supernatants was measured by ELISA (Fig. 1B). The treatment with TRAIL and TNF-α caused a significant increase in PGE₂ in the supernatants of COX-2 overexpressing cells U-87MG and MG-377 compared with each control (p<0.01), and addition of NS-398 into their cultures significantly reduced it (p<0.01). Although PGE₂ was detectable in the supernatant of LN-18, there was no significant difference in each group.

### EXAMPLE 11

### DCs Efficiently Phagocytose Apoptotic Glioma Cells

Apoptotic death was analyzed using Annexin V-FITC Apoptosis Detection Kit and FACScan where Ann V⁺/ Pr cells indicate early stage of apoptosis and Ann V⁺/PI⁺ cells also indicate dead cells. The treatment of glioma cells with TRAIL and TNF-α caused a significant increase in the ratio of apoptotic death (p<0.01) and Ann V⁺/PI⁺ death (p<0.01) compared with the control in each group (Fig. 2A). The treatment of iDCs with TRAIL and TNF-α did not induce their death (data not shown). NS-398 treatment did not cause a change of the death ratio in each glioma (data not shown).

Glioma cells were dyed red with PKH-26 before co-culturing with iDCs. DCs were stained with anti-CD11c-FITC antibody after 16 hours co-culture, and isolated CD11c+ DCs were analyzed by FACScan where double positive cells indicate DCs phagocytosed glioma cells. It has been shown that DCs can phagocytose apoptotic cells by the recognition of phosphatidylserine which can be detected by Annexin V (Albert, M.L. et al., "Dendritic cells acquire antigen from apoptotic cells and induce class I-restricted CTLs," Nature, Vol. 392, pp. 86-89 (1998); Albert, M.L. et al., "Immature dendritic cells phagocytose apoptotic cells via alphavbeta5 and CD36, and cross-present antigens to cytotoxic T lymphocytes," J. Exp. Med., Vol. 188, pp. 1359-1368 (1998)). In fact, a significant increase of Ann V⁺ death in glioma caused a significant increase (p<0.01) in the ratio of DCs which phagocytosed glioma cells compared with each control (Fig. 2B).

### EXAMPLE 12

### DCs Can Be Matured by The Treatment with TNF-α after Phagocytosis of Glioma Cells

DCs were stained with anti-CD86, HLA-DR, CD83, CCR7, and CD11c antibody. CD11c+ DCs were isolated with cell separation unit and microbease-conjugated anti-FITC antibody. Single-cultured iDCs were highly expressing CD86 and HLA-DR, but did not express CD83 or CCR7 which are maturation markers of DCs (Fig. 3). The co-stimulation of TRAIL and/or PGE₂ on single-cultured iDCs up-regulated CD86 expression (geometric mean; iDCs:647, TRAIL-DCs:1086, PGE₂-DCs:2779, TRAIL/PGE₂-DCs:1176), but they did not induce CD83 or CCR7 expression (Fig. 3). The co-stimulation with TNF-α on single-cultured iDCs induced both CD83 and CCR7 expressions, and co-stimulation with TNF-α and PGE₂ strongly up-regulated these maturation markers (Fig. 3). These data indicate that stimulation of TNF-α is required for maturation of immature DCs.

Expression of CD86 on DCs containing glioma cells was significantly down-regulated compared with single-cultured iDCs (geometric mean; co-cultured DCs: 317-480, single cultured DCs: 647-4720; p< 0.01), but there was no significant difference on the ratio of CD86 positive cells (co-cultured DCs: 95-98%, single cultured DCs: 96-98%) (Fig. 3). Importantly, both CD83 and CCR7 expressions on co-cultured DCs were highly expressed as well as single-cultured TRAIL/ TNF-α /PGE₂-DCs (Fig. 3). There was no statistical difference on geometric means of the expressing markers in the variation of co-cultured glioma cells or NS-398 treatment (Fig. 3). These data indicate that DCs can be completely matured by the effect of TNF-α stimulation, even if they contained apoptotic glioma cells.

### EXAMPLE 13

### Inhibition of COX-2 Protein in Gliomas Is Effective in IL-10 Reduction and IL-12p70 Augmentation in Tumor-infiltrating DCs

IL-10 and IL-12p70 in the supernatants of cultures were measured by ELISA, and the results were statistically compared with NS-398(-) single-cultured DCs (Fig. 4). COX-2 inhibitor for single-cultured DCs did not effect to change IL-10 or IL-12p70 production. U-87MG (p<0.01), LN-18 (p<0.05) and MG-377 (p<0.05) affected the co-cultured DCs to enhance IL-10, and also U-87MG (p<0.01) and LN18 (p<0.05) affected to suppress IL-12p70. COX-2 inhibition in gliomas remedied the IL-10 surplus and IL-12 deficiency in the co-cultured DCs. There was no detectable IL-10 or IL-12p70 in the supernatants of glioma cell cultures (data not shown). These data indicate that COX-2 expression in glioma cells relates to IL-10 augmentation and/or IL-12 reduction in co-cultured DCs.

### EXAMPLE 14

### COX-2 Inhibitor in Glioma Has a Capacity for DCs-mediating Th Polarization to Bias Toward Th1

MLR by co-culturing with CD4+ and CD8+ lymphocytes and autologous DCs was performed, and Th cell polarization was confirmed by ELISPOT assay (Fig. 5A-F). The results were statistically compared with the lymphocyte which was initially-stimulated by NS-398(-) single-cultured DCs. The DCs co-cultured with glioma cells induced tumor-specific Th response which was polarized toward Th2, and inhibition of COX-2 in gliomas resulted in polarization toward Th1 (Fig. 5A-D).

The DCs co-cultured with autologous tumor (MG-377) did not change the tumor-specific Th response compared with their single-cultured DCs (Fig. 5E, F), indicating that glioma patients already had a similar Th response against their tumors which could be induced by the tumor-infiltrating DCs. However, this Th response was polarized toward Th2. The DCs co-cultured with COX-2-inhibited glioma cells changed Th polarization toward Th1 (Fig. 5E, F). These data indicate that COX-2 inhibitor in glioma has a capacity for DC-mediated Th polarization to bias toward Th1.

## Claims

1. A cyclooxygenase (COX)-2 inhibitor for use in the treatment of a cancer in a human, wherein the treatment includes the administration of a dendritic cell (DC) vaccine to said human.

2. The COX-2 inhibitor for use in claim 1 wherein the cancer is **characterized by** overexpression of COX-2.

3. The COX-2 inhibitor for use in claim 1 or 2, wherein the cancer is a glioma.

4. The COX-2 inhibitor for use in any one of claims I to 3, wherein the DC vaccine comprises from about 10⁵ to about 10⁷ DC.

5. The COX-2 inhibitor for use in any one of claims 1 to 3, wherein the COX-2 inhibitor is provided in a dose of from 0.1 to 10,000 mg per day.

6. The COX-2 inhibitor for use in any one of claims 1 to 3, wherein the COX-2 inhibitor is provided in a dose of from 1.0 to 1,000 mg per day.

7. The COX-2 inhibitor for use in claim 1 or 2, wherein the cancer is selected from the group consisting of lung cancer, bladder cancer, colorectal cancer, and brain cancer.

8. The COX-2 inhibitor for use in claim 1 or 2 wherein the cancer is a brain cancer.

9. The COX-2 inhibitor for use in claim 1 or 2, wherein said cancer is selected from the group consisting of glioma, astrocytomas, ependymal tumors, glioblastoma multiforme, and primitive neuroectodermal tumors.

10. The COX-2 inhibitor for use in any one of the preceding claims, wherein said COX-2 inhibitor is NS-398, celecoxib, rofecoxib, valdecoxib, or meloxicam.

11. The COX-2 for use in inhibitor according to any one of the preceding claims, wherein the COX-2 inhibitor is present in an amount sufficient to selectively inhibit Prostaglandin E₂ (PGE₂) activity in the human.

12. A cyclooxygenase (COX)-2 inhibitor and dendritic cells (DCs) for use in the treatment of a cancer in a human.

13. The COX-2 inhibitor for use in claim 12, wherein the cancer is **characterized by** overexpression of COX-2.

14. The COX-2 inhibitor for use in claim 12 or 13, wherein the cancer is a glioma.

## Patentansprüche

1. Cyclooxygenase-(COX)-2-Inhibitor zur Verwendung in der Behandlung eines Krebses bei einem Menschen, wobei die Behandlung die Verabreichung eines Impfstoffs mit dendritischen Zellen (DC) an den Menschen einschließt.

2. COX-2-Inhibitor zur Verwendung in Anspruch 1, wobei der Krebs durch Überexpression von COX-2 gekennzeichnet ist.

3. COX-2-Inhibitor zur Verwendung in Anspruch 1 oder 2, wobei der Krebs ein Gliom ist.

4. COX-2-Inhibitor zur Verwendung in einem der Ansprüche 1 bis 3, wobei der DC-Impfstoff von etwa 10⁵ bis etwa 10⁷ DC umfasst.

5. COX-2-Inhibitor zur Verwendung in einem der Ansprüche 1 bis 3, wobei der COX-2-Inhibitor in einer Dosis von 0,1 bis 10000 mg pro Tag bereitgestellt wird.

6. COX-2-Inhibitor zur Verwendung in einem der Ansprüche 1 bis 3, wobei der COX-2-Inhibitor in einer Dosis von 1,0 bis 1000 mg pro Tag bereitgestellt wird.

7. COX-2-Inhibitor zur Verwendung in Anspruch 1 oder 2, wobei der Krebs aus der Gruppe, bestehend aus Lungenkrebs, Blasenkrebs, kolorektalem Krebs und Gehimkrebs, ausgewählt ist.

8. COX-2-Inhibitor zur Verwendung in Anspruch 1 oder 2, wobei der Krebs ein Gehimkrebs ist.

9. COX-2-Inhibitor zur Verwendung in Anspruch 1 oder 2, wobei der Krebs aus der Gruppe, bestehend aus Gliom, Astrozytomen, Ependymtumoren, Glioblastoma multiforme und primitiven neuroektodermalen Tumoren, ausgewählt ist.

10. COX-2-Inhibitor zur Verwendung in einem der vorhergehenden Ansprüche, wobei der COX-2-Inhibitor NS-398, Celecoxib, Rofecoxib, Valdecoxib oder Meloxicam ist.

11. COX-2 zur Verwendung im Inhibitor gemäß einem der vorhergehenden Anspruch, wobei der COX-2-Inhibitor in einer Menge, ausreichend, um selektiv Prostaglandin-E₁-(PGE₂)-Aktivität bei dem Menschen zu hemmen, vorhanden ist.

12. Cyclooxygenase-(COX)-2-Inhibitor und dendritische Zellen (DCs) zur Verwendung in der Behandlung eines Krebses bei einem Menschen.

13. COX-2-Inhibitor zur Verwendung in Anspruch 12, wobei der Krebs durch Überexpression von COX-2 gekennzeichnet ist.

14. COX-2-Inhibitor zur Verwendung in Anspruch 12 oder 13, wobei der Krebs ein Gliom ist.

## Revendications

1. Inhibiteur de cyclooxygénase (COX)-2 pour une utilisation dans le traitement d'un cancer chez un humain, dans lequel le traitement inclut l'administration d'un vaccin de cellules dendritiques (DC) audit humain.

2. Inhibiteur de COX-2 pour une utilisation dans la revendication 1, dans lequel le cancer est **caractérisé par** une surexpression de COX-2.

3. Inhibiteur de COX-2 pour une utilisation dans la revendication 1 ou 2, dans lequel le cancer est un gliome.

4. Inhibiteur de COX-2 pour une utilisation dans l'une quelconque des revendications 1 à 3, dans lequel le vaccin DC comprend d'environ 10⁵ à environ 10⁷ DC.

5. Inhibiteur de COX-2 pour une utilisation dans l'une quelconque des revendications 1 à 3, où l'inhibiteur de COX-2 est fourni dans une dose de 0,1 à 10.000 mg par jour.

6. Inhibiteur de COX-2 pour une utilisation dans l'une quelconque des revendications 1 à 3, où l'inhibiteur de COX-2 est fourni dans une dose de 1,0 à 1.000 mg par jour.

7. Inhibiteur de COX-2 pour une utilisation dans la revendication 1 ou 2, dans lequel le cancer est choisi dans le groupe constitué d'un cancer du poumon, d'un cancer de la vessie, d'un cancer colorectal et d'un cancer du cerveau.

8. Inhibiteur de COX-2 pour une utilisation dans la revendication 1 ou 2, dans lequel le cancer est un cancer du cerveau.

9. Inhibiteur de COX-2 pour une utilisation dans la revendication 1 ou 2, dans lequel ledit cancer est choisi dans le groupe constitué d'un gliome, d'astrocytomes, de tumeurs épendymaires, d'un glioblastome multiforme et de tumeurs neuroectodermiques primitives.

10. Inhibiteur de COX-2 pour une utilisation dans l'une quelconque des revendications précédentes, où ledit inhibiteur de COX-2 est le NS-398, le célécoxib, le rofécoxib, le valdécoxib ou le méloxicam.

11. COX-2 pour une utilisation dans un inhibiteur selon l'une quelconque des revendications précédentes, où l'inhibiteur de COX-2 est présent dans une quantité suffisante pour inhiber sélectivement l'activité de la Prostaglandine E₂ (PEG₂) chez l'humain.

12. Inhibiteur de cyclooxygénase (COX)-2 et cellules dendritiques (DCs) pour une utilisation dans le traitement d'un cancer chez un humain.

13. Inhibiteur de COX-2 pour une utilisation dans la revendication 12, dans lequel le cancer est **caractérisé par** une surexpression de COX-2.

14. Inhibiteur de COX-2 pour une utilisation dans la revendication 12 ou 13, dans lequel le cancer est un gliome.
